# EUROPEAN PATENT APPLICATION

(11) **EP 2 620 109 A1**
(43) Date of publication of application: **31.07.2013**
(21) Application number: 13152784.8
(22) Date of filing: 25.01.2013
(51) Int. Cl.: A61B 17/22, A61B 19/00, A61N 2/12, A61M 25/01, A61B 17/00, A61B 17/3207

(54) **Magnetic clot disrupter**

(30) Priority: 27.01.2012 US 201261591658 P
(71) Applicant: Cook Medical Technologies LLC, Bloomington, IN 47404 (US)
(72) Inventor: Tekulve, Kurt J., Ellettsville, IN Indiana 47429 (US)
(74) Representative: Garratt, Peter Douglas

(57) **Abstract**

A magnetic wire and medical procedure are provided. The wire is used to disrupt a clot within a body vessel. The wire includes a magnet that is positioned within the patient's body adjacent the clot. A magnetic driver is positioned outside the patient's body in proximity to the magnet. When the magnetic field of the magnetic driver is alternated, the wire moves within the body vessel to break up the clot.

## Description

### BACKGROUND

The present invention relates generally to medical devices and more particularly to a device to disrupt a clot in a body vessel.

One medical condition that some patients suffer from his deep vein thrombosis (DVT). DVT most commonly affects the veins in the legs and can occur for a number of reasons. Typically, DVT occurs in patients with a reduced rate of blood flow or physiological conditions that make a patient's blood more susceptible to clotting. Although DVT can be uncomfortable and painful for patients due to swelling and decreased blood circulation in a patient's leg, DVT is particularly dangerous because it is possible for large blood clots to break loose from the blockage and flow through the venous system to the lungs. This can result in a pulmonary embolism, which can be fatal in certain cases.

Typically, DVT conditions can be identified early by observing the swelling and discomfort symptoms that often accompany DVT. Early conditions of DVT may be referred to as acute DVT, and in this stage, DVT is characterized by a buildup of clotted blood. In contrast, chronic DVT is characterized by the development of fibrous tissue that may become a more permanent blockage of the vein.

Commonly, DVT is treated with the use of thrombolytic drugs, such as tPA, which chemically dissolves clots. DVT has also been treated with intraluminal devices that rotate within the vessel to break up clots. Filters are also used downstream of a clotted region to catch any clots that break loose before the clots reach the lungs.

Although various methods currently exist for treating DVT, the inventor believes that an improved method and medical device would be desirable for treating DVT and possibly other conditions.

### SUMMARY

A magnetic wire and medical procedure are described for disrupting a clot in a body vessel. The wire has a magnet attached to a core. The magnet is disposed along a distal portion of the wire intended to be inserted into a patient's body vessel. The magnet is positioned adjacent a clot in the body vessel, and an exterior magnetic driver is positioned in proximity to the magnet. By alternating the magnetic field of the magnetic driver, the magnet and wire are moved laterally within the body vessel to disrupt the clot. The inventions herein may also include any other aspect described below in the written description, the claims, or in the attached drawings and any combinations thereof.

### BRIEF DESCRIPTION OF SEVERAL VIEWS OF THE DRAWINGS

The invention may be more fully understood by reading the following description in conjunction with the drawings, in which:

Figure 1 is a cross-sectional view of a wire for disrupting a clot;

Figure 2 is a cross-sectional view of another wire for disrupting a clot;

Figure 3 is a cross-sectional view of another wire for disrupting a clot; and

Figure 4 is a cross-sectional view of a body vessel with a wire inserted through the body vessel to disrupt a clot.

### DETAILED DESCRIPTION

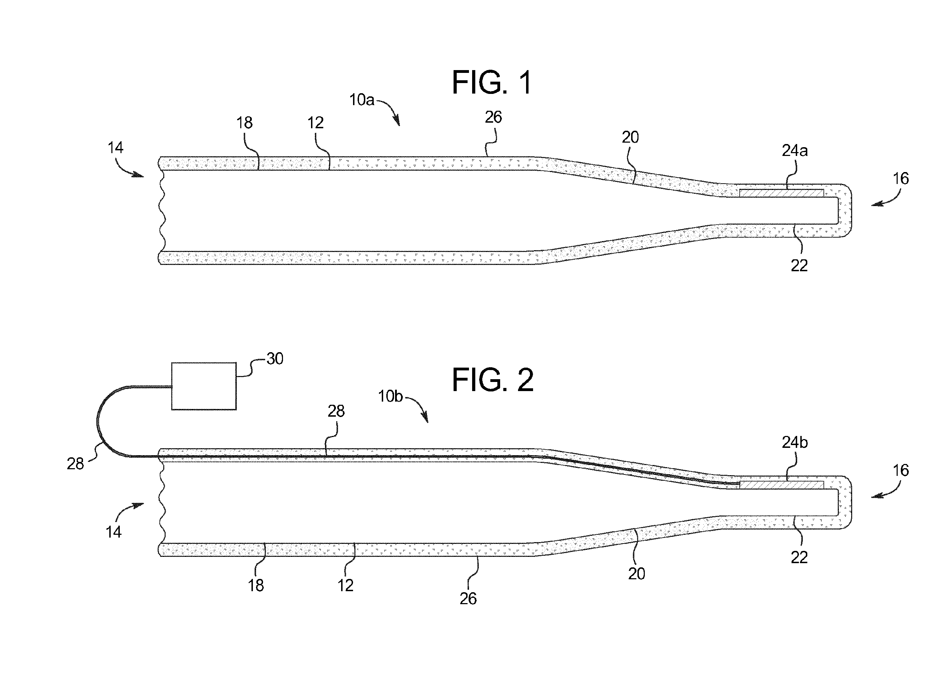

Referring now to the figures, and particularly to Figure 1, a wire 10a for disrupting a clot 32 in a body vessel 34 is shown. The wire 10a includes a core 12 that preferably extends from the proximal end 14 to the distal end 16 of the wire 10a. The overall length of the wire 10a is long enough so that the distal end 16 of the wire 10a extends through a patient's internal anatomy 34 to reach the desired treatment site where a clot 32 is located. For example, the overall length of the wire 10a may be about 140 cm to about 180 cm. The proximal end 14 of the wire 10a is intended to remain outside the patient's body 48 and may be manipulated by a physician to move the wire 10a through the patient's anatomy 34. Preferably, the core 12 has a proximal portion 18 with a larger cross-sectional profile adapted to transmit pushing and torque forces to move the wire 10a through the patient's anatomy 34. The core 12 may also include a tapered portion 28 toward the distal end 16 of the wire 10a where less rigidity in the wire 10a is desired. Preferably, the tapered portion 20 is about 8 mm to about 16 mm in length. As further described below, the tapered portion 20 may transition to a distal portion 22 of the core 12 with a smaller cross-sectional profile than the proximal portion 18. The cross-sectional area of the distal portion 22 of the core may be between 25% and 75% or less than or equal to 80%, 70%, 60%, 50%, 40%, 30% or 20% of the cross-sectional area of the proximal portion 18 of the core or of that part of the proximal portion 18 of the core that is adjacent the distal portion 22 or the tapered portion 20 of the core. As a result, the distal portion 22 is more flexible than the proximal portion 18. The length of the more flexible distal portion may be less than or equal to 5 mm, 10 mm, 20 mm or 30 mm. Although the core 12 may be formed as a solid wire, the core 12 may also be formed of a helical wire structure or may be a multi-component structure. One preferred material for the core 12 is nitinol.

The wire 10a also includes a magnet 24a disposed along the distal portion 22 of the wire 10a. Various types of magnets 24 may be used with the wire 10, but in the embodiment of Figure 1, a permanent magnet 24a is preferred. The magnet 24a is also preferably embedded within a polymer layer 26 so that the entire magnet 24a is encased on the core 12 by the polymer layer 26. The polymer layer 26 may also cover the entire portion of the core 12 that is intended to be inserted into a patient's anatomy 34. Various types of polymers 26 that are biocompatible may be used, such as polyurethane, PTFE or nylon.

Turning to Figure 2, another embodiment of the wire 10b is shown. In Figure 2, the wire 10b may have a magnet 24b that is an electromagnet 24b instead of a permanent magnet 24a. As shown, the wire 10b may include an electrical conductor 28 that is connected to the magnet 24b and to an electrical source 30 that would typically be located outside the patient's body 48. The conductor 28 extends along the wire 10b, and preferably, is encased within the polymer layer 26. Thus, in Figure 2, the magnet 24b may be magnetized only during the medical treatment as further described below instead of being permanently magnetized as in Figure 1. The polymer layer 26 may also serve to insulate the conductor 28.

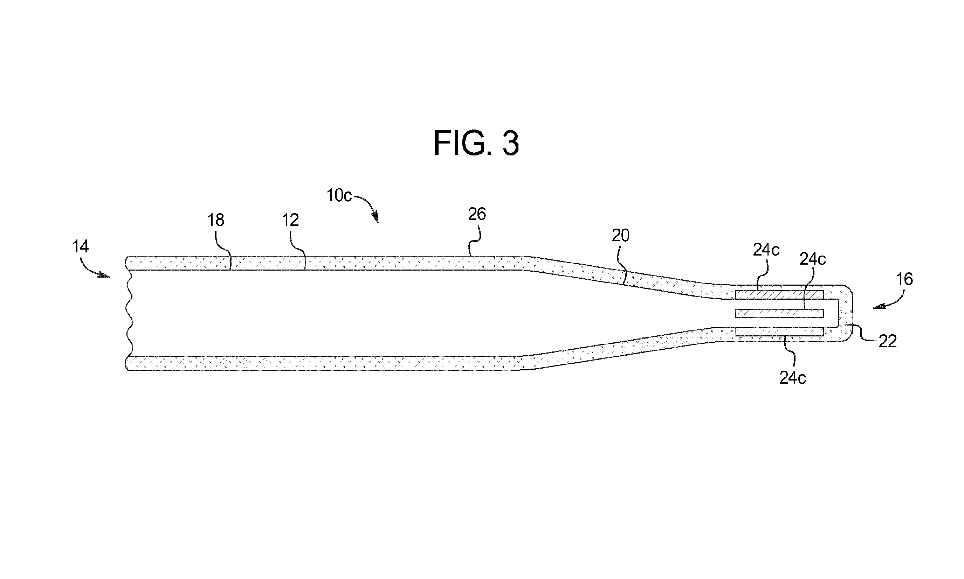

Turning to Figure 3, the wire 10c may also have multiple magnets 24c disposed around the circumference of the distal portion 22 of the wire 10c. The number and orientation of the magnets 24c may be altered as desired in order to achieve the desired wire movement 10c in response to the magnetic source 30 as described below. Further, the magnets 24c may be electrically connected to separate conductors 28, as described above, in order to magnetize each of the magnets at different times during the procedure if desired.

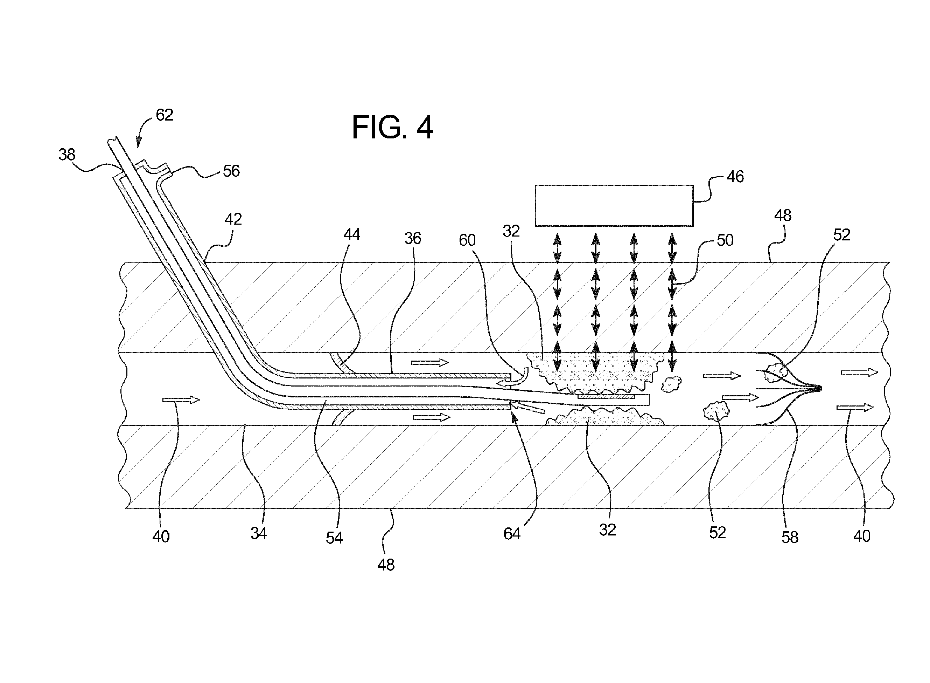

Turning to Figure 4, a medical procedure is shown that may be used for disrupting a clot 32, such as blood clots 32 in deep vein thrombosis (DVT). In the procedure, the wire 10 is inserted through a vein 34 until the magnet 24 is adjacent the clot 32 in the vessel 34. The wire 10 may be inserted through a sheath 36 as shown in Figure 3, or alternatively, the wire 10 may be used without a sheath 36. If a sheath 36 is used, the sheath 36 may be provided with a seal 38 that the wire 10 may be inserted through, and once the wire 10 and sheath 36 are positioned at the treatment site, the seal 38 may be tightened onto the wire 10 to prevent leakage through the seal 38. Preferably, where the wire 10 is inserted through a vein 34, the wire 10 is inserted through the vein 34 along the direction of blood flow 40, with the distal end 16 of the wire 10 being downstream from the access site 42. Thus, the wire 10 passes through the valves 44 of the vein 34 in the direction that the valves 44 are intended to open in order to minimize damage to the valves 44.

Once the magnet 24 is positioned adjacent the clot 32, a magnetic driver 46 is positioned outside the patient's body 48 in a location proximate to the magnet 24. A magnetic field 50 is then generated by the magnetic driver 46 so that the magnet 24 is attracted to and/or repelled from the magnetic driver 46. The magnetic field 50 may be alternated by repeatedly turning the field 50 on and off or by reversing the charge of the field 50. As a result, the magnet 24 and the portion of the wire 10 that the magnet 24 is connected to are repeatedly drawn toward the magnetic driver 46 and/or pushed away from the magnetic driver 46. This causes the wire 10 to repeatedly press laterally into the clot 32, which causes the clot 32 to break up. Preferably, the distal portion 22 of the core 12 that the magnet 24 is attached to has a smaller cross-sectional profile to make the distal portion 22 more flexible than the proximal portion 18 so that the magnet 24 readily moves laterally within the vessel 34 in response to the magnetic driver 46. In use of a wire comprising an electromagnet, for example as shown in Figure 2, it may alternatively or additionally be possible to interrupt or alternate the current supplied to the electromagnet in the wire, to effect lateral movement of the distal portion of the wire with the vessel. In another embodiment, the magnet 24 may comprise a body of magnetisable material, although this is less preferred.

The broken up clots 52 may be dissolved using thrombolytic drugs, which may be injected through the annular space 54 between the wire 10 and the sheath 36. For example, the sheath 36 may be provided with a port 56 outside the patient's body 48 where the drugs may be injected into the sheath 36. A filter 58 may also be released in the vessel 34 at a location downstream from the magnet 24 and clot 32. Thus, when the clots 52 break loose, they are caught by the filter 58 before reaching vital organs. The clots 52 that are caught by the filter 58 may then be dissolved by thrombolytic drugs; may dissolve naturally due to the body's physiological processes; may be mechanically disrupted further by the filter 58; or may be removed from the body 48 by removing the filter 58. The broken clots 52 may also be collected by the sheath 36 by applying suction 60 to the annular space 54 between the sheath 36 and the wire 10. Negative pressure 60 may be applied by exerting suction 60 at the port 56 on the proximal end 62 of the sheath 36. Therefore, when positioning the sheath 36 in the vessel 34, it is desirable to locate the distal end 64 of the sheath 36 as close as possible to the clot 32 in order to exert suction pressure 60 onto the clot 32 and to release thrombolytic drugs directly at the clot 32.

After the magnetic driver 46 has been used to drive the wire 10 into the clot 32 to break up the clot 32, it may be desirable to rotate the magnetic driver 46 around the patient's body 48 and alternate the current again. This will cause the wire 10 to move laterally in the vessel 34 in a different plane from the first use of the magnetic driver 46. That is, because the wire 10 primarily moves along a plane toward and/or away from the magnetic driver 46, it may be useful to change the position of the magnetic driver 46 multiple times in order to drive the wire 10 along different lateral planes. For example, it may be desirable to rotate the magnetic driver 46 at least 60° from the first location to drive the wire 10 along a second plane. It may also be desirable to rotate the magnetic driver 46 about 90° from the first plane or other increments less than 60°. Alternatively, multiple magnetic drivers 46 may be used, either in separate steps or simultaneously.

One advantage of the wire 10 for use in disrupting clots 32 is the small profile of the wire 10 and the simplicity of the wire 10 itself. This may minimize trauma to the patient, and also, particularly to the valves 44 in a patient's vein 34. While the method does require an external magnetic driver 46 to force lateral movement of the wire 10 within the patient's vessel 34, the magnetic driver 46 may be a reusable piece of equipment in the physician's office. Thus, the single-use components are limited to the wire 10 and the sheath 36 and/or filter 58. In addition, physicians may find the procedure simpler and more desirable than other devices once physicians become comfortable with the use of an exterior magnetic driver 46 to move a wire 10 within a vessel 34 for the purpose of breaking up clots 32. While the wire 10 may be used in other medical procedures, it is believed that the wire 10 may be especially useful in treating deep vein thrombosis, and particularly, for treating acute clots 32 before a clot 32 has developed significant fibrous tissue or other hardened structures. Chronic clots and other obstructions may benefit less from the described method because such structures typically involve a more integrated and solid structure that is less amenable to being broken up by the lateral movement expected from the described method.

While preferred embodiments of the invention have been described, it should be understood that the invention is not so limited, and modifications may be made without departing from the invention. The scope of the invention is defined by the appended claims, and all devices that come within the meaning of the claims, either literally or by equivalence, are intended to be embraced therein. Furthermore, the advantages described above are not necessarily the only advantages of the invention, and it is not necessarily expected that all of the described advantages will be achieved with every embodiment of the invention.

## Claims

1. A system for disrupting a clot within a body vessel, comprising:
a wire insertable through a body vessel such that a proximal end of said wire extends outside a patient's body and a distal end of said wire extends inside said patient's body, said wire comprising a magnet disposed along a distal portion of said wire to be disposed within said patient's body such that
said magnet can be positioned adjacent a clot within said body vessel;
a magnetic driver adapted to be positioned in proximity to said magnet outside of said patient's body; and
means for alternating a magnetic field generated by said magnetic driver or said magnet;
such that in use said magnetic field forces said magnet and a portion of said wire to move laterally within said vessel, said wire thereby repeatedly pressing into said clot for breaking up said clot.

2. The system according to claim 1, further comprising a filter releasable within said body vessel, said filter being disposable downstream from said magnet and said clot, wherein said filter in use catches portions of said clot broken by said wire.

3. The system according to claim 1 or claim 2, further comprising a sheath, said wire extending in use through said sheath such that a distal end of said sheath may be disposed proximal from said clot, the system being adapted for and preferably comprising means for suction to be applied to said sheath as said magnetic field is alternated, portions of said clot broken by said wire thereby being collected in use by said sheath.

4. The system according to any one of the preceding claims, wherein said wire comprises a core, said core having a smaller cross-sectional profile along said distal portion of said wire than along a proximal portion of said wire, said proximal portion of said wire thereby being adapted to transmit higher pushing and torque forces than said distal portion, and said distal portion being more flexible than said proximal portion.

5. The system according to any one of the preceding claims, wherein said magnet is embedded within a polymer layer.

6. The system according to any one of the preceding claims, wherein said magnet is a permanent magnet.

7. The system according to any one of claim 1 to claim 5, further comprising a conductor extending along said wire from said magnet and connected to an electrical source, said magnet thereby being an electromagnet.

8. The system according to any one of the preceding claims, wherein said magnetic driver is configured to be rotated with respect to a patient's body at least 60° to force in use said magnet in another plane of movement.

9. The system according to any one of the preceding claims, wherein said magnetic field can be alternated by turning said field on and off, said magnet thereby in use being repeatedly drawn toward said magnetic driver and returning its unforced position.

10. The system according to any one of claim 1 to claim 8, wherein said magnetic field can be alternated by reversing said field, said magnet thereby in use being repeatedly drawn toward said magnetic driver and pushed away from said magnetic driver.

11. A wire for use in a system for disrupting a clot within a body vessel, comprising:
a core with a distal end adapted to extend inside a patient's body through a body vessel and a proximal end adapted to extend outside said patient's body; and
a magnet disposed at or along a distal portion of said core adapted to be disposed within said patient's body;
wherein said magnet is moveable within said body vessel by a magnetic driver disposed in proximity to said magnet outside of said patient's body to break up a clot within said body vessel.

12. The wire according to claim 11, wherein said magnet is embedded within a polymer layer.

13. The wire according to claim 11 or claim 12, wherein said distal portion of said core along which said magnet is disposed has a smaller cross-sectional profile than a proximal portion of said core, said proximal portion of said core thereby being adapted to transmit higher pushing and torque forces than said distal portion, and said distal portion being more flexible than said proximal portion.

14. The wire according to claim 11, claim 12 or claim 13, wherein said magnet is a permanent magnet.

15. The wire according to claim 11, claim 12 or claim 13, further comprising a conductor extending along said core from said magnet to a location adapted to connect to an electrical source, said magnet thereby being an electromagnet.
